# EUROPEAN PATENT SPECIFICATION

(11) **EP 4 054 411 B1**
(45) Date of publication and mention of the grant of the patent: **12.03.2025**
(21) Application number: 20796787.8
(22) Date of filing: 22.10.2020
(51) Int. Cl.: G01N 21/67, A61B 5/08, A61B 5/00, A61B 5/097, G01N 33/497

(54) **EXHALED GAS ANALYSIS**
ANALYSE VON AUSGEATMETEM GAS
ANALYSE DE GAZ EXHALÉ

(30) Priority: 07.11.2019 US 201916677333
(43) Date of publication of application: 14.09.2022
(73) Proprietor: Respiro B.V., 2312 NV Leiden (NL)
(72) Inventor: VORKOV, Vitalii, 1079 LP Amsterdam (NL)
(74) Representative: Arnold & Siedsma
(86) International application number: PCT/EP2020/079769
(87) International publication number: WO 2021/089338

(56) References cited:
- EP-A1- 0 556 614
- WO-A2-2004/058064
- US-A- 3 951 607
- US-A- 5 078 494
- US-A1- 2016 054 294

## Description

### FIELD OF THE INVENTION

The invention relates to a spectral analysis of gases exhaled by a patient and, more particularly, to a universal portable breath content analyzer and a method of exhaled gas analysis based on the use of the aforementioned analyzer.

### DESCRIPTION OF THE PRIOR ART

Analysis of gases exhaled by a patient refers to non-invasive methods for diagnosing patients, which causes increased interest.

It is known that the content of air exhaled from a human (or animal) body is directly associated with biochemical and physiological processes that occur in the body. It is also known that one of the most important factors in the existence of living organisms is their gas exchange with the environment. The basis of this gas exchange is absorption of oxygen and the release of water vapor and carbonic acid gas, which occur during external respiration and are mainly due to the energy consumption of the body. These processes are so intense that changes in the concentration of oxygen (O₂) and carbon dioxide (CO₂) due to respiration reach several percent (> 3%) of the total composition of exhaled air. These and other light gaseous compounds, which are formed in much smaller amounts in the body, are present in the exhaled air in the form of traces (concentration of less than 10-6%) and are signs or markers of ongoing biochemical processes. Data on the release of such substances and their concentration are valuable for the diagnosis of certain diseases. In other words, gases in the exhalation of a patient may be associated with predetermined diseases. For example, provision of acetone (C₃H₆O) in an exhale sample in an amount of 4 to 20 ppm may characterize a pancreas function at acute destructive pancreatitis, dietary imbalance, severe heart failure, or lung cancer. A presence of methanol (CH₃OH) or ethanol (C₂H₆O) in an amount of > 500 ppm or acetaldehyde (CH₃CHO) in an amount of 4 - 20 ppm may be associated with diseases of the central nervous system, sugar diabetes, or alcoholism, etc.

Determination of micro composition in the exhaled gas is one of the most difficult analytical tasks. Only few physicochemical methods for determining trace amounts of gaseous substances were found in the art. Among them are gas chromatography (GC), mass spectrometry combined with gas chromatographic separation (MS-GC), electrochemical sensors (SEC), semiconductor sensors (PS), UV chemoluminescence (UVCL), and IR spectroscopy (IR). The last includes Fourier transform spectroscopy, optoacoustic spectroscopy (OAS), and laser spectroscopy (LS).

Heretofore, many breath content analyzers and methods of breath content analysis were developed, used in a medical diagnostic practice, and disclosed in the scientific and patent literature. A big group of such devices and methods relate to spectral analysis of gas samples exhaled by a patient.

For example, US Patent No. 7153272 issued on December 26, 2006 to Talton discloses methods of collecting and detecting compounds in a human breath sample. The method consists of the steps of exhaling into a handheld sample collector to absorb at least one breath compound in an exhaled breath collector, connecting the handheld sample collector to a breath analyzer, transferring the breath compounds from the exhaled breath collector into the breath analyzer, and detecting breath compounds using two or more sensors. The method may be performed to detect breath compounds for determining health or disease diagnosis, or for drug monitoring. detection may be performed using mass spectroscopy, or electronic, optical, or acoustic vapor sensors. Sensors may include at least one sensor selected from the group consisting of surface acoustic wave sensors, shear horizontal wave sensors, flexural plate wave sensors, quartz microbalance sensors, conducting polymer sensors, dye-impregnated polymer film on fiber optic detectors, conductive composite sensors, chemiresistors, metal oxide gas sensors, electrochemical gas detectors, chemically sensitive field-effect transistors, and carbon black-polymer composite devices. The sensors are removable and/or replaceable. A breath sample may comprise multiple breath compounds, including, but not limited to, alcohols, ethers, ketones, amines, aldehydes, carbonyls, carbanions, alkanes, alkenes, alkynes, aromatic hydrocarbons, polynuclear aromatics, biomolecules, sugars, isoprenes, isoprenoids, indoles, pyridines, fatty acids, and off-gases of a microorganism.

US Patent No. US 6955652 issued on October 18, 2005 to Baum, et al. discloses a non-invasive, miniature, breath monitoring apparatus for spectroscopic multi-component breath monitoring and analysis. The system is comprised of one or more IR emitters focused by optical elements through a low volume sample cell receiving a sample input of a patient's breath for analysis. The patient either at rest or during exercise, inhales C₂H₂-SF₆ mixtures (balance of oxygen and nitrogen) which is subsequently monitored upon exhalation for CO₂, H₂O, C₂H₂, and SF₆, which can be employed to determine Q (the amount of blood pumped by the heart per minute) directly and accurately. Measurements are performed in real-time or via post-processing of stored original data. The miniature analyzer operates on the principle of infrared absorption spectroscopy.

US Patent No. S5095913 issued on March 17, 1992 to Yelderman, et al. discloses methods and apparatus for constructing optically stabilized, shutterless infrared capnographs. The capnographs provide absolute concentrations of the constituents of a patient's respiratory airstream without thermal drift problems normally associated with thermopile detectors, thereby providing a device with a high degree of accuracy. The present invention eliminates the need for a mechanical shutter to modulate the incident infrared beam and the need for a modulated source, thereby increasing the reliability and response time of the devices disclosed. Capnographs, which are substantially unaffected by changes in the ambient temperature at which they operate, are provided by connecting pairs of optically filtered thermopiles in series and processing the resulting differential pairs.

US Patent No. 6469303 issued on October 22, 2002 to Sun, et al. discloses a non-dispersive infrared sensor that includes a cylindrical metallic tube, a printed circuit board platform that fits into one end of the tube, a diffusion filter that fits into the opposite end of the tube, and an optical system. The optical system includes an infrared source on the platform, a mirror on the inner wall of the tube so as to reflect and focus the infrared light from the infrared source, and a detector assembly that receives the infrared light after reflection. The gas sensor may further include a partition between the infrared source and the detector assembly, a removable filter on the diffusion filter, connecting pins attached to the platform, and a sealing layer formed under the platform. The detector assembly includes a signal detector and a reference detector. A first and second bandpass filters are respectively formed on the signal and reference detectors.

US Patent Application Publication No. 20170146449 issued May 25, 2017 (Inventor: Coates) discloses a multi-component gas and vapor monitoring sensor device that contains a series of optical spectral sensors for gas and vapor measurements using a combination of solid-state light sources (LED or Broadband) and multi-element detectors, housed within an integrated package that includes the interfacing optics and acquisition and processing electronics. Spectral selectivity is provided by a custom detector eliminating the need for expensive spectral selection components. The multi-component gas monitor system has no moving parts, and a gas sample flows through a measurement chamber where it interacts with a light beam created from the light source, such as a MEMS broad band IR source or a matrix of LEDs. A custom detector(s) is/are configured with multi-wavelength detection to detect and measure the light beam as it passes through the sample within the measurement chamber.

US Patent No. 5800360 issued on September 1, 1998 to Kisner, et al. discloses a passive, non-invasive, non-contacting apparatus and method for monitoring the respiration of a subject within a monitored environment. The apparatus generally comprises a pair of sensors, which detect changes in infrared energy. The first sensor detects changes in infrared energy, which signifies and corresponds to changes in the monitored environments of a component to be monitored and generates the first signal. The second sensor detects changes in infrared energy, which signifies reference infrared energy in the monitored environment and generates the second signal. A processing system converts the first and second signals into a third signal, which signifies the concentration of the monitored component in the monitored environment. The monitored components may be carbon dioxide (CO₂), water vapor (H₂O) or a constituent of exhaled breath such as a ketone, amino acid, insulin or pintane. In another embodiment, changes in blood pH may be monitored by adding an additional sensor. Micromotion of the subject's body may also be monitored in yet another embodiment through the use of a single sensor together with an appropriate processing system. Imaging techniques may be employed to accomplish high resolution monitoring of the monitored environment.

US Patent No. 5747809 issued on May 5, 1998 to Eckstrom discloses an NDIR apparatus and method for measuring isotopic ratios in gaseous samples. The apparatus provides four separate optical paths for separate measurement of each of two isotopes relative to a reference signal, using spectrally resolved infrared radiation. The design permits the measurements to be made accurately without significant time lags between measurements, and without interchanging of cells or filters.

Trace amounts of infrared active gases, such as CO₂, CO, NOₓ, or CH₄, can be routinely detected by non-dispersive infrared spectroscopy. The filtered IR radiation passes through a gas sample, where it is absorbed in proportion to the amount of that species present, and falls on a detector, which measures the fraction of radiation transmitted. Alternatively, the radiation may be filtered after passing through the gas sample. An unattenuated reference signal may also be generated by including a filter, which restricts radiation to a range where no absorption occurs.

US Patent No. 5693944 issued on December 2, 1997 to Rich discloses monitoring the level of carbon dioxide in the breath of a medical patient. This is typically done during a surgical procedure as an indication to the anesthesiologist of the patient's condition. As the patient's wellbeing, and even his or her life, is at stake, it is of paramount importance that the carbon dioxide concentration be measured with great accuracy.

The gas analyzer system includes: (1) a transducer for outputting a signal indicative of the concentration of a specified gas in a sample, which may contain that gas, and (2) an airway adapter or cuvette with a flow passage for confining the sample to a particular path traversing the transducer. The cuvettes feature radiant energy transmitting windows, which are flush mounted in apertures on opposite sides of the cuvette flow passage and are fabricated from a polymer such as biaxially oriented polypropylene which is malleable, yet resistant to wrinkling, warping, and other forms of distortion. Retainer rings keep the windows flat and distortion free with an accurately reproducible spacing between the windows.

US5078494A discloses a glow discharge emission spectrometer for breath analysis comprising a vacuum pump, pressure transducer, control of pressure in the gas chamber, etc. A first electrode and a counter electrode are both at least partly located inside the sealed housing.

Another breath gas analyzer based on spectroscopy is known from US2016/054294A1, comprising an amplifying mirror arrangement.

### SUMMARY OF THE INVENTION

The invention relates to a spectral analysis of gases exhaled by a patient and, more particularly, to a universal portable breath content analyzer and a method of exhaled gas analysis based on the use of the aforementioned analyzer.

The device of the invention has a sealed tubular housing. From one end, the housing is connected to an air evacuation tube, which, in turn, is connected to a vacuum pump and from the opposite side to a high-voltage source and an expiratory sampling mouthpiece. In accordance with one aspect of the invention, the housing is made from a purified fused quartz or suprasil glass. From the outer side, the tubular housing according to the invention is encompassed by a semi-cylindrical mirror. On the side opposite to the mirror, the tubular housing has an opening. Inserted into this opening is a flat transparent glass plate made from the same material as the tubular housing. The glass plate supports a replaceable optical filter assembly, which contains a number of flat optical filters, each being intended for filtering out lights of a predetermined bandwidth. The optical filter assembly supports a sensor or a group of sensors. The optical sensor assembly consists of a plurality, e.g., four optical sensors. The sensor assembly is replaceable together with the appropriate optical filters, and each individual sensor operates in conjunction with a respective optical filter. The sensors are connected to a Central Processing Unit (hereinafter CPU), which may be comprised of a personal computer, a tablet, or even a smart phone with a special software or App. The same CPU controls operation of valves in the system of evacuation of air from the tubular housing and taking of a sample of breath from a patient via another valve. A pressure sensor is provided for measuring pressure in the tubular housing. The pressure sensor is connected to the CPU, which controls operation of the valves in a manner that maintains the pressure in the housing at a constant level during measurement.

The mirror is intended for reflecting a dissipated energy of the luminescent light generated by a glow discharge, which is generated in the tubular housing under effect of the voltage applied to the interior of a pre-evacuated tubular housing. Generation of the discharge in a predetermined synchronization with the evacuation of air and apply of a voltage of predetermined level is performed under control of the CPU.

### BRIEF DESCRIPTION OF DRAWINGS

Fig. 1 is a longitudinal sectional view that shows main parts of the device of the invention.
Fig. 2 is a cross-section along line II-II of Fig. 1.
Fig. 3 a schematic sectional view of the entire system according to one aspect of the device of the invention that illustrates interconnection of the device with components of the control system.
Fig. 4 is an exploded three-dimensional view of the replaceable optical waveband filter assembly.
Fig, 5 is a block diagram of an entire breath content analyzer which is not in accordance with the invention.
Fig. 6A and 6B are examples of spectra obtained with the use of a spectrometer in a sample of an exhaled gas associated with a glow discharge, wherein Fig. 6A is for 20 ppm concentration of acetone in the exhaled test probe and Fig. 6B for 100 ppm concentration of acetone in the exhaled test probe.

### DETAILED DESCRIPTION OF THE INVENTION

The invention relates to a spectral analysis of gases exhaled by a patient and, more particularly, to a universal portable breath content analyzer and a method of exhaled gas analysis based on the use of the aforementioned analyzer.

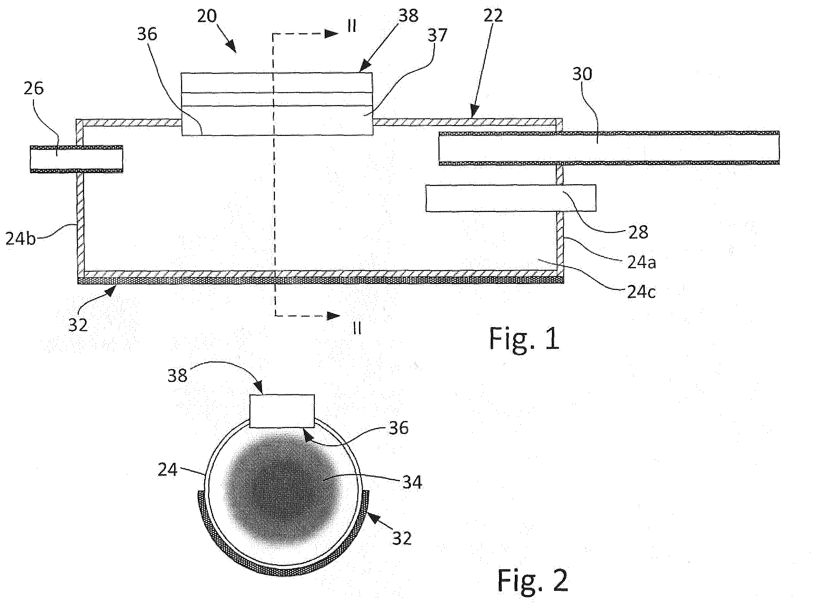

A universal portable breath content analyzer (herein after referred to as a "device" or "analyzer") according to one aspect of the invention is shown in Figs. 1 and 2, wherein Fig. 1 is a longitudinal sectional view of the device, and Fig. 2 is a cross-section along line II-II of Fig. 1.

As shown in Figs. 1 and 2, the device, which in an assembled state is designated by reference numeral 20, has a sealed tubular housing 22, which consists of a cylindrical body 24 and end-face wall 24a and 24b. Let us conventionally call the face-end wall 24a a front wall and the face-end wall 24b a rear wall.

Through the rear wall 24b, an interior 24c of the sealed housing 22, which is defined by the outer wall, in this case the cylindrical body 24, the front end wall 24a, and the rear end face wall 24b, is connected to an air evacuation tube 26, which, in turn, is connected to a vacuum pump (not shown in Figs. 1 and 2) and through an electrode 28 that passes through the front wall 24a to a high-voltage source (not shown in Figs. 1 and 2).

A respiratory sampling mouthpiece 30 through which a sample of an air exhaled from a patient is taken also passes into the interior 24c of the sealed tubular housing 22 through the front wall 24a.

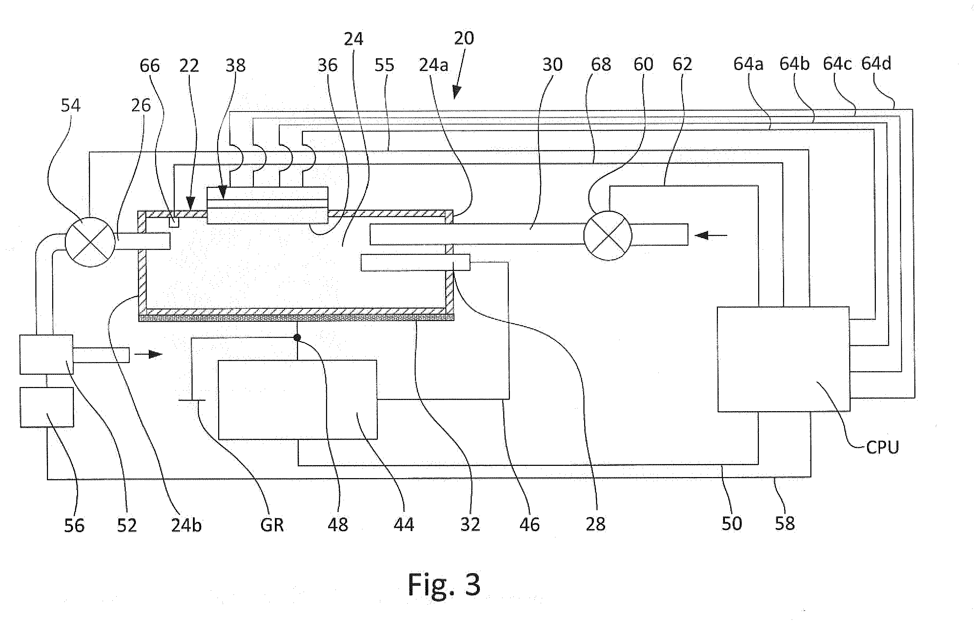

According to the invention, at least a part of the outer wall is transparent, and the counter electrode is a mirror that is located outside the outer housing and encompasses said at least a part of the outer wall, which is transparent. In the modification shown in Figs. 1 and 2, the entire sealed tubular housing 22 is made, e.g., from a purified fused silica or suprasil glass. From the outer side, the sealed tubular housing 22 is encompassed by a semi-cylindrical mirror 32 (Fig. 2). The mirror is intended for amplifying an output signal, which is generated by molecules of gases that are present in the interior 24c of the sealed tubular housing 22 and activated by a discharge, e.g., a glow discharge 34 induced in the tubular housing 22. Fig. 3 is a schematic sectional view of the entire system of the device 20 of the invention that illustrates interconnection of the device 20 with components of the control system. In the modification of Fig. 3, the mirror 32 is used as a counter electrode relative to the electrode 28 located on the high-voltage source side.

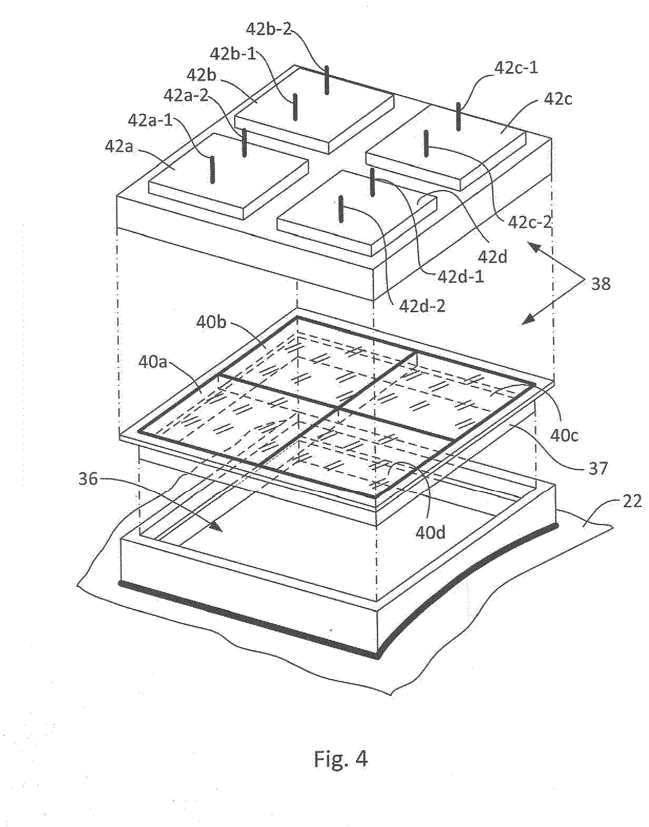

On the side opposite to the mirror 32, the tubular housing 22 has an opening 36. Inserted into the opening 36 is a flat transparent glass plate 37 made from the same material as the tubular housing 22. The glass plate 36 supports a set of replaceable optical filter/waveband sensor assemblies, hereinafter referred to as replaceable optical filter assemblies 38, which contains a number of flat optical bandpass filters 40a, 40b, 40c, and 40d that are shown in Fig. 4, which is an exploded three-dimensional view of the replaceable optical waveband filter assembly 38. Four optical waveband filters are shown only as an example and the number of the optical filters may be different. Herein, each bandwidth filter is intended for filtering out lights of a predetermined bandwidth. It is well known that molecules of different excited gases emit lights of different wavelengths, which are used for identification of the presence of respective gaseous components, in this case, in the respiratory gases exhaled by a patient into the interior 24 of the sealed tubular housing 22 through the respiratory sampling mouthpiece 30.

The optical filter assembly 38 supports sensors 42a, 42b, 42c, and 42d, which are aligned with the respective optical bandwidth filters 40a, 40b, 40c, and 40d and intended for receiving optical signals from the respective filters and for converting these optical signals into electrical signals that correspond to the gas components contained in the respiratory gas sampled and analyzed by the device 20. The following sensors/photodiodes can be used for UV wavelengths GaP photodiodes (e.g. FGAP71 from Thorlabs), for visual and in the beginning of infrared range wavelengths Si photodiodes (e.g. FDS010 from Thorlabs), and for near-infrared wavelengths InGaAs photodiodes (e.g. FD10D from Thorlabs). However, particular sensors are determined by the particular task with regard to measured gas components.

The optical sensors 42a, 42b, 42c, and 42d are parts of the optical filter assembly 38 and are replaceable together with the filters as an integral unit. In other words, the breath content analyzer 20 of the invention may contain a set of such replaceable filter assemblies 38 for covering different bandwidth ranges. Since in visible and near-infrared light spectra the light-emitting molecules present in a gas discharge show more than one line of illumination, the use of the aforementioned set of replaceable optical filter assemblies 38 makes it possible to expand an assortment of gas components to be identified and thus increase versatility of the breath content analyzer 20 of the invention.

In the modification of Fig.3, the mirror 32, as an electrode, is grounded at GR. The mirror 32 is also connected to the high-voltage power source 44 via a link 48. The high-voltage power source 44 is connected to the CPU via a link 50. An example of a high-voltage power source is E15 by EMCO which can deliver up to 1500V with power of 3W.

In Fig. 4, reference numerals 42a-1, 42a-2, 42b-1, 42b-2 ..... 42d-1, 42d-2 designate electrical terminals, from which the component-identifying electric signals of the sensors are sent to a central processing unit, hereinafter CPU 40, shown in Fig. 3. The CPU 40 may be comprised of a personal computer, a tablet, or even a smart phone with a special software or App.

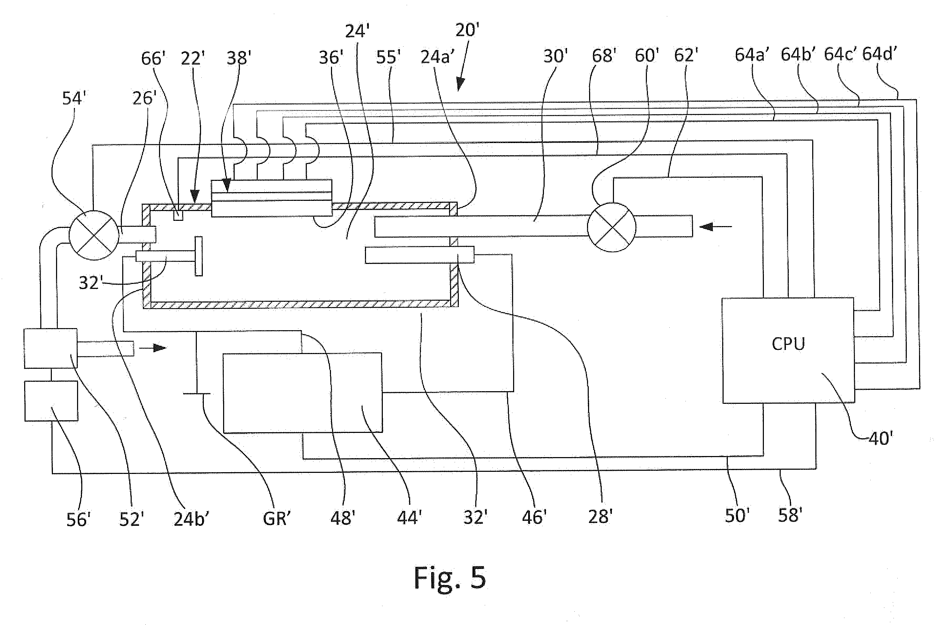

In addition to the parts and assemblies mentioned above, an analyzer 20' contains some other important parts and components, which are shown in the modification of Fig. 5. This modification, which is not according to the invention, differs from those according to the invention shown in Figs. 1 to 4 in that a rod-like counter electrode 32' that passes into the interior 24 of the housing 22 through the rear wall 24b is used instead of the mirror 32. In Fig. 5, those parts and assemblies that were described earlier will be designated by the same numeral references as in Figs. 1 to 4 but with addition of a prime ('). Thus, in Fig. 5 the housing is designated by reference numeral 22', the electrode 28 is designated by reference numeral 28', etc. As can be seen from Fig. 5, in addition to the sealed tubular housing 22', air evacuation tube 26', electrode 28', CPU 40', respiratory sampling mouthpiece 30', flat transparent glass plate 36', and the replaceable optical waveband filter assembly 38', the analyzer 20' is also equipped with other important elements, which have not been described above. Among them is a high voltage power source 44', which is connected to the electrode 28' via a link 46'. It is understood that both electrodes have opposite polarities. Reference numeral 52' designates a vacuum pump, which evacuates air from the interior 24' of the sealed tubular housing 22' via a cut-off valve 54' of the air evacuation system. The cut-off valve 54' is connected to the CPU 40' via a link 55'. The vacuum pump 52' is controlled by a driver 56', which is connected to the CPU via a link 58'.

A flow control valve 60' that ensures a metered gas flow into the vacuum system is installed on the inlet end of the respiratory sampling mouthpiece 30'. The valve 60' is also linked to the CPU 40' via a link 62'.

As has been mentioned above with reference to Fig. 3, the electrical terminals 42a-1, 42a-2, 42b-1, 42b-2 ..... 42d-1, 42d-2 of the respective sensors 42a, 42b, 42c, and 42c are linked to the CPU 40'. In Fig. 5, these links are designated by reference numerals 64a', 64b,' 64c', and 64d'. Installed in the interior 24 of the sealed tubular housing 22 is a pressure sensor 66, which is linked to the CPU via a link 68. As will be shown below, a provision of the pressure sensor 66 for measuring the pressure of gas in the interior 24' of the housing 22' is a factor very important for realization of a method according to which at all measurements of the breath components the pressure is maintained at a constant level regardless of the expiratory volume produced by the patient. Accomplishment of this condition is absolutely necessary for obtaining quantitative data on the content of the sought components, which are necessary for the subsequent data analysis and diagnostics.

Let us consider the operation of the device 20 of the invention in accordance with the first modification shown in Fig. 3.

First a pressure that has to be maintained in the interior 24 is pre-assigned in the CPU 40. The valve 60 is shut off, and the air contained in the interior of the housing 22 is evacuated via the valve 54 by the vacuum pump 52. After evacuation, the valve 54 is shut off, the valve 60 is open, and the patient exhales a portion of air into the interior 24 of the housing 22 via the valve 60. The gas evacuation valve 54 remains closed, the pressure inside the housing is controlled by the pressure sensor 66, and when a given pressure at which all measurements are conducted is achieved the valve 60 is closed.

A voltage of about 300V-5000V is then applied to the electrode 28, and a glow discharge 34 (Fig. 2) is generated in the interior 24 of the tubular housing between the electrodes, i.e., the mirror 32 and the electrode 28. Conditions for generation of the glow discharge in the analyzer of a specific geometry are provided by precondition data inputted to the CPU with reference to the specific dimensions, inter-electrode distance, level of vacuum in the interior 24 of the housing 22, type of the gas, etc.

Since the tubular housing 22 is transparent, the portion of light incident onto the mirror 32 is reflected back to the glow discharge whereby the signal of the luminescent light of glow discharge is intensified.

Through the glass plate 37 and the respective optical bandwidth filters 40a, 40b, 40c, and 40d, the light passes to sensors 42a, 42b, 42c, and 42d that convert the optical signals into electric signals, which are then sent to the CPU from their terminals 42a-1, 42a-2, 42b-1, 42b-2, 42c-1, 42c-2, 42d-1, and 42d-2 via respective lines 64a, 64b, 64c, and 64d.

Upon completion of the measurement, both valves 60 and 54 are opened, and the interior 24 of the cylindrical housing 22 is scavenged by evacuating the exhaled air probe from the housing for the preparation of the device to the next breath analysis cycle.

The analyzer 20' of the second modification shown in Fig.5, which is not according to the invention, works in the same manner as the analyzer 20 except that a metal counter electrode 32' is used instead of a mirror-type electrode 32 of the previous modification.

For the modification of Fig. 5, not according to the invention, the mirror is not needed.

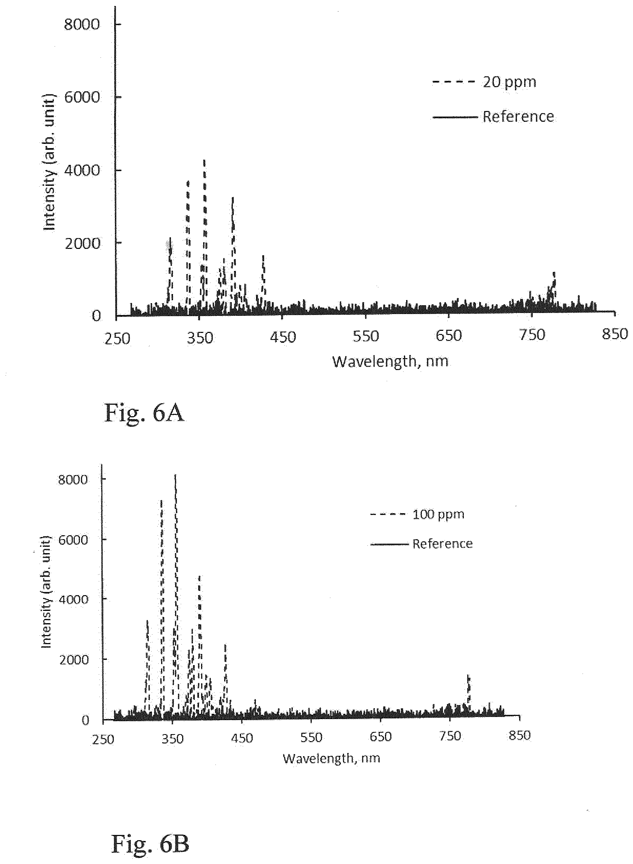

Fig. 6A and 6B are examples of spectra obtained with the use of a spectrometer in samples of an exhaled gas associated with a glow discharge, wherein Fig. 6A is for 20 ppm concentration of acetone in the exhaled test probe, and Fig. 6B for 100 ppm concentration of acetone in the exhaled test probe.

These spectrograms also show that intensity of the lines of the spectra depends on a partial concentration of the acetone in the exhaled gas. This fact is used in calibration of the breath content analyzer 20 of the invention.

In two considered examples the following intensity values in arbitrary units have been obtained for four considered characteristic lines: 20 ppm case: Line 1- 3650.9, Line 2 - 4230.9, Line 3 - 3231.9, Line 4 - 1079.0; 100 ppm case: Line 1- 7376.4, Line 2 - 8147.4, Line 3 - 4853.4, Line 4 - 1250.4. These data can be used further in order to correlate concentration of acetone with the measured intensity of characteristic lines.

It is understood that main parameters of the optical bandpass filters 40a, 40b, 40c, and 40d are bands of transparency for respective lines of luminescence. Each filter passes only one line of predetermined wavelength. Such an approach makes it possible to reveal and identify specific gas components present in the gas mixture, in this case, an exhaled gas sample. The search for components other than those associated with an appropriate group of filters will require replacement of the present kit of filter-sensor assemblies 38 (Fig. 1 and Fig. 3). As mentioned above, the analyzer 20 of the invention contains a set of such replaceable filter assemblies for covering different bandwidth ranges. On the other hand, dependence of the intensity of luminescence from concentration of the sought gas component makes it possible to evaluate the presence of this component quantitatively.

The analyzer 20 (20') may operate in different modes. Let us consider as an example the operation of the analyzer system, which is not in accordance with the invention, shown in Fig. 5. First, with the flow control valve 60' being shut off, the valve 54' is open, and air is evacuated from the interior of the housing 22' by activating the pump 52'. The pressure inside the housing 22' is controlled by the CPU 40' via the pressure sensor 66'. When a predetermined pressure optimal from the viewpoint of obtaining the most reliable measurement results is achieved, the valve 54' is shut off, the mouthpiece 30' is inserted into the patient's mouth (not shown), the flow control valve 60' is opened, a sample of an exhaled air is admitted into the housing 22', and when the pressure in the housing reaches a predetermined value, the valve 60' is shut off. Simultaneously with shutting off the valve 60', a high voltage is applied to the electrode 28' from the high-voltage power source 44'. As a result, a glow discharge 34 of the type shown in Fig. 2 is generated inside the housing 22' between the electrode 28' and the counter electrode 24'. The light of the discharge is transmitted through the glass plate 36' and the filters 40a, 40b, 40c, and 40d to the respective sensors 42a', 42b', 42c' , and 42d'. The sensors 42a', 42b', 42c, and 42d', which receive the discharge emission light that passed through the corresponding filters generate electrical signals the amplitudes of which are proportional to the concentration of the sought components. These signals are transmitted via the respective links 64a', 64b', 64c', and 64d' to the CPU' 40, where the obtained data are analyzed.

A method of exhaled gas analysis conducted under a constant pressure will be described with reference the modification of Fig. 5, which is a modification not according to the invention. The method consists of providing an exhaled gas analysis device 20 (20') of the type described above. Next step is closing the flow control valve 60', opening the shut off valve 54', evacuating air from the sealed housing 22', closing the shut off valve 54' upon completion of air evacuation, opening the flow control valve 60', admitting an air inhaled from a patient into the sealed housing 22' while measuring the pressure inside the sealed housing 22', closing the flow control valve 60' when the pressure measured by the pressure sensor 66' reaches a value of the given constant value, generating a discharge in the sealed housing 22', passing a light produced by the discharge through optical filters 40a, 40b, 40c, and 40d (Fig. 4) that pass the light of predetermined bandwidth in proportion to the content of components contained in the exhaled air, detecting the content of the components; and conducting spectral analysis of components contained in the air exhaled by the patient into the sealed housing. Upon the completion of the detecting the content of the components, the interior of the sealed housing can be scavenged
The analyzer of the present invention has the following essential distinctions from conventional devices of this class:
1) It is intended for operation, i.e., for taking the breath sample, i.e., in a mode of constant pressure. This makes it possible to obtain quantitative data and conduct quantitative analysis of components present in an exhaled gas for use in disease diagnostics. This is achieved by maintaining a pressure in the device housing 22 at a desired level due to a provision of a pressure sensor 66 nside the housing 22 and the CPU-controlled inlet and outlet valves 60 and 54 of the housing. In this manner it is possible to select a pressure for the light emission optimal from the viewpoint of obtaining meaningful results. Conventional breath analyzers with permanent evacuation of gas from the analyzer housing are subject to considerable variations in the volume of the test gas since the volumes of exhale from different patients may vary almost in the range of 100%. Thus, quantitative evaluation of the exhale gas content becomes practically impossible.
2) The material and construction of the analyzer housing are selected to improve sensitivity of the analysis.
3) Provision of replaceable sets of filter-sensor assemblies 38 for different wavelength bands makes it possible to match the sensor assemblies with specific emission lines that correspond to specific component contents.
4) The analyzer features mentioned above make it possible to diagnose various diseases and determine a degree of their severity.
5) The design and parts from which the analyzer is built make it possible to embody it in a form of a small portable device having dimensions in the range of 25 and 50 mm for length, 40 and 70 mm for width, and 100 and 250 mm for height.

Although the invention was described and illustrated in detail using the preferred example embodiments, the invention is not restricted to the examples disclosed and other variations can be derived by a person skilled in the art without departing from the scope of protection of the invention as defined by the appended claims.

For example, not only a glow discharge can be used for activation of the emission from the sought components. Sensors may be comprise a system of bandwidth filters applied one onto the other.

## Claims

1. A universal portable breath content analyzer (20) comprising:
a sealed housing (22) having an outer wall, a first end face wall (24a) and a second end face wall (24b) located opposite to the first end face, the sealed housing (22) having an interior defined by the outer wall, the first end face, and the second end face;
a first electrode (28) that passes into the interior of the sealed housing (22) through one of the end faces;
a voltage supply source connected to the first electrode (28) for applying a voltage to the first electrode;
a counter electrode (32) for interaction with the first electrode (28) and for generating a discharge in the interior of the sealed housing (22) when a voltage sufficient for generating a discharge between the first electrode (28) and the second electrode (32) is applied to the first electrode;
a mouthpiece (30) for taking a sample of an exhaled air exhaled by a patient and for supplying the exhaled air into the interior of the sealed housing (22);
a flow control valve (60) installed in the mouthpiece (30) for keeping the mouthpiece (30) open or closed;
an air evacuation tube (26) inserted into the interior of the sealed housing (22);
a vacuum pump (52) with a driver (56) connected to the air evacuation tube (26);
a shut-off valve (54) installed in the air evacuation tube (26) between the interior of the sealed housing (22) and the vacuum pump (52);
a pressure sensor (66) located inside the interior of the sealed housing (22) for measuring pressure in said interior;
an opening (36) in the outer wall;
a transparent plate (37) installed in the opening (36);
a set of replaceable optical filter/waveband sensor assemblies (38) removably installable onto the transparent plate (37), each replaceable optical filter/waveband filter assembly comprising a waveband filter for passing light of the discharge having a predetermined waveband and a sensor capable of converting optical signals of the discharge into electrical signals; and
central processing unit (40), which is connected to the flow control valve, the shut-off valve, the voltage supply source, the driver (56) of the vacuum pump (52), and the pressure sensor (66) of each replaceable optical filter/waveband filter assembly for controlling operations of aforementioned devices depending on the pressure measured by the pressure sensor (66),
wherein the housing (22) is a cylindrical body, at least a part of the outer wall is transparent, and the counter electrode (32) is a semi-cylindrical mirror that is located outside the outer housing (22) and encompasses said at least a part of the outer wall, which is transparent.

2. The universal portable breath content analyzer (20) according to Claim 1, wherein the CPU is selected from the group consisting of a personal computer, a tablet, and a smart phone.

3. The universal portable breath content analyzer (20) according to Claim 1, wherein CPU is selected from the group consisting of a personal computer, a tablet, a smart phone.

4. A method of exhaled gas analysis conducted under a constant pressure comprising:
a) providing an exhaled gas analysis device comprising a sealed housing (22), a pressure sensor (66) for measuring pressure in the sealed housing (22), a flow control valve (60) for controlling admission of exhaled air from a patient into the sealed housing (22), a shut off valve connected to a vacuum pump (52) for evacuation of air from the sealed housing (22), the sealed housing (22) having an outer wall, a first end face wall (24a) and a second end face wall (24b) located opposite to the first end face, the sealed housing (22) having an interior defined by the outer wall, the first end face, and the second end face, a first electrode (28) that passes into the interior of the sealed housing (22) through one of the end faces, and a counter electrode (32) for interaction with the first electrode (28) and for generating a discharge in the interior of the sealed housing (22) when a voltage sufficient for generating a discharge between the first electrode (28) and the second electrode (32) is applied to the first electrode, and wherein the housing (22) is a cylindrical body, at least a part of the outer wall is transparent, and the counter electrode (32) is a semi-cylindrical mirror that is located outside the outer housing (22) and encompasses said at least a part of the outer wall, which is transparent;
b) closing the flow control valve;
c) opening (36) the shut off valve and evacuating air from the sealed housing (22);
d) closing the shut off valve upon completion of air evacuation; opening (36) the flow control valve;
e) admitting an air inhaled from a patient into the sealed housing (22) while measuring the pressure inside the sealed housing (22);
f) closing the flow control valve (60) when the pressure measured by the pressure sensor (66) reaches a value of said constant pressure;
g) generating a discharge in the sealed housing (22);
h) passing a light produced by said discharge through optical filters that pass the light of predetermined bandwidth in proportion to the content of components contained in the exhaled air;
i) detecting the content of the components; and
j) conducting spectral analysis of components contained in the air exhaled by the patient into the sealed housing (22).

5. The method according to Claim 4, further providing the exhaled gas analysis device with a central processing unit (40); connecting the central processing unit (40) to the flow control valve, the shut-off valve, and the pressure sensor (66) for controlling operation of the flow control valve, the shut-off valve, and pressure sensor (66), maintaining the pressure inside the sealed housing (22) during said step of detecting the content of the components; and sending the content of the components to the central processing unit (40).

6. The method according to Claim 4, further comprising scavenging the sealed housing (22) upon completion of at least the steps from a) to j).

7. The method according to Claim 5, further comprising scavenging the sealed housing (22) upon completion of at least the steps from a) to j).

8. The method according to Claim 4, wherein the steps g) and h) are conducted by providing the exhaled gas analysis device with a set of replaceable optical filter/waveband sensor assemblies (38) removably installable in the sealed housing (22) in a position capable of executing the steps h) and i).

9. The method according to Claim 5, wherein the steps h) and i) are conducted by providing the exhaled gas analysis device with a set of replaceable optical filter/waveband sensor assemblies (38) removably installable in the sealed housing (22) in a position capable of executing the steps h) and i).

10. The method according to Claim 6, wherein the steps g) and h) are conducted by providing the exhaled gas analysis device with a set of replaceable optical filter/waveband sensor assemblies (38) removably installable in the sealed housing (22) in a position capable of executing the steps h) and i).

11. The method according to Claim 7, wherein the steps h) and i) are conducted by providing the exhaled gas analysis device with a set replaceable optical filter/waveband sensor assemblies (38) removably installable in the sealed housing (22) in a position capable of executing the steps g) and h).

## Patentansprüche

1. Universeller tragbarer Ateminhaltsanalysator (20), umfassend:
ein abgedichtetes Gehäuse (22), das eine Außenwand, eine erste Endflächenwand (24a) und eine zweite Endflächenwand (24b), die sich gegenüber von der ersten Endflächenwand befindet, aufweist, wobei das abgedichtete Gehäuse (22) einen Innenraum aufweist, der durch die Außenwand, die erste Endfläche und die zweite Endfläche definiert ist;
eine erste Elektrode (28), die in den Innenraum des abgedichteten Gehäuses (22) durch eine der Endflächen übergeht;
eine Spannungsversorgungsquelle, die mit der ersten Elektrode (28) zum Anlegen einer Spannung an die erste Elektrode verbunden ist;
eine Gegenelektrode (32) für eine Wechselwirkung mit der ersten Elektrode (28) und zum Generieren einer Entladung in dem Innenraum des abgedichteten Gehäuses (22), wenn eine Spannung, die zum Generieren einer Entladung zwischen der ersten Elektrode (28) und der zweiten Elektrode (32) ausreicht, an die erste Elektrode angelegt wird;
ein Mundstück (30) zum Entnehmen einer Probe von ausgeatmeter Luft von einem Patienten und zum Zuführen der ausgeatmeten Luft in den Innenraum des abgedichteten Gehäuses (22);
ein Durchflussregelventil (60), das in dem Mundstück (30) installiert ist, um das Mundstück (30) offen oder geschlossen zu halten;
ein Luftableitungsröhrchen (26), das in den Innenraum des abgedichteten Gehäuses (22) eingesetzt ist;
eine Vakuumpumpe (52) mit einem Antrieb (56), der mit dem Luftableitungsröhrchen (26) verbunden ist;
ein Absperrventil (54), das in dem Luftableitungsröhrchen (26) zwischen dem Innenraum des abgedichteten Gehäuses (22) und der Vakuumpumpe (52) installiert ist;
einen Drucksensor (66), der sich im Inneren des Innenraums des abgedichteten Gehäuses (22) zum Messen von Druck in dem Innenraum befindet;
eine Öffnung (36) in der Außenwand;
eine transparente Platte (37), die in der Öffnung (36) installiert ist;
einen Satz auswechselbarer optischer Filter/Wellenbandsensor-Baugruppen (38), die auf der transparenten Platte (37) entfernbar installierbar sind, jede auswechselbare optische Filter/Wellenbandfilter-Baugruppe umfassend einen Wellenbandfilter zum Durchlassen von Licht der Entladung mit einem vorbestimmten Wellenband und einen Sensor, der in der Lage ist, optische Signale der Entladung in elektrische Signale umzuwandeln; und
eine zentrale Verarbeitungseinheit (40), die mit dem Durchflussregelventil, dem Absperrventil, der Spannungsversorgungsquelle, dem Antrieb (56) der Vakuumpumpe (52) und dem Drucksensor (66) jeder austauschbaren optischen Filter-/Wellenbandfilter-Baugruppe verbunden ist, zum Regeln des Betriebs der vorgenannten Vorrichtungen in Abhängigkeit von dem Druck, der durch den Drucksensor (66) gemessen wird,
wobei das Gehäuse (22) ein zylindrischer Körper ist, mindestens ein Teil der Außenwand transparent ist und die Gegenelektrode (32) ein halbzylindrischer Spiegel ist, der sich außerhalb des Außengehäuses (22) befindet und mindestens den einen Teil der Außenwand umgibt, der transparent ist.

2. Universeller tragbarer Ateminhaltsanalysator (20) nach Anspruch 1, wobei die CPU aus der Gruppe ausgewählt ist, bestehend aus einem Personalcomputer, einem Tablet und einem Smartphone.

3. Universeller tragbarer Ateminhaltsanalysator (20) nach Anspruch 1, wobei die CPU aus der Gruppe ausgewählt ist, bestehend aus einem Personalcomputer, einem Tablet und einem Smartphone.

4. Verfahren für die Analyse von ausgeatmetem Gas, das unter konstantem Druck vorgenommen wird, umfassend:
a) Bereitstellen einer Vorrichtung für die Analyse von ausgeatmetem Gas, umfassend ein abgedichtetes Gehäuse (22), einen Drucksensor (66) zum Messen des Drucks in dem abgedichteten Gehäuse (22), ein Durchflussregelventil (60) zum Regeln des Einlasses von ausgeatmeter Luft eines Patienten in das abgedichtete Gehäuse (22), ein Absperrventil, das mit einer Vakuumpumpe (52) für die Ableitung von Luft aus dem abgedichteten Gehäuse (22) verbunden ist, wobei das abgedichtete Gehäuse (22) eine Außenwand, eine erste Endflächenwand (24a) und zweite Endflächenwand (24b), die sich gegenüber von der ersten Endfläche befindet, aufweist, wobei das abgedichtete Gehäuse (22) einen Innenraum, der durch die Außenwand, die erste Endfläche und die zweite Endfläche definiert ist, eine erste Elektrode (28), die in den Innenraum des abgedichteten Gehäuses (22) durch eine der Endflächen übergeht, und eine Gegenelektrode (32) für die Wechselwirkung mit der ersten Elektrode (28) und zum Generieren einer Entladung in dem Innenraum des abgedichteten Gehäuses (22), wenn eine Spannung, die zum Generieren einer Entladung zwischen der ersten Elektrode (28) und der zweiten Elektrode (32) ausreicht, an die erste Elektrode angelegt wird, aufweist und wobei das Gehäuse (22) ein zylindrischer Körper ist, mindestens ein Teil der Außenwand transparent ist, und die Gegenelektrode (32) ein halbzylindrischer Spiegel ist, der sich außerhalb des äußeren Gehäuses (22) befindet und mindestens einen Teil der Außenwand umgibt, die transparent ist;
b) Schließen des Durchflussregelventils;
c) Öffnen (36) des Absperrventils und Ableiten von Luft aus dem abgedichteten Gehäuse (22);
d) Schließen des Absperrventils nach Abschluss des Luftableitens; Öffnen (36) des Durchflussregelventils;
e) Einlassen von eingeatmeter Luft von einem Patienten in das abgedichtete Gehäuse (22), während eines Messens des Drucks im Inneren des abgedichteten Gehäuses (22);
f) Schließen des Durchflussregelventils (60), wenn der Druck, der durch den Drucksensor (66) gemessen wird, einen Wert des konstanten Drucks erreicht;
g) Generieren einer Entladung in dem abgedichteten Gehäuse (22);
h) Leiten eines Lichts, das durch die Entladung durch optische Filter erzeugt wird, die das Licht einer vorbestimmten Bandbreite entsprechend dem Inhalt von Komponenten, die in der ausgeatmeten Luft enthalten sind, durchleitet;
i) Erkennen des Inhalts der Komponenten; und
j) Vornehmen einer Spektralanalyse von Komponenten, die in der ausgeatmeten Luft von dem Patienten in das abgedichtete Gehäuse (22) enthalten sind.

5. Verfahren nach Anspruch 4, wobei ferner die Vorrichtung für die Analyse von ausgeatmetem Gas mit einer zentralen Verarbeitungseinheit (40) versehen ist; Verbinden der zentralen Verarbeitungseinheit (40) mit dem Durchflussregelventil, dem Absperrventil und dem Drucksensor (66) zum Regeln des Betriebs des Durchflussregelventils, des Absperrventils und des Drucksensors (66), wobei der Druck im Inneren des abgedichteten Gehäuses (22) während des Schritts des Erkennens des Inhalts der Komponenten aufrechterhalten wird; und Senden des Inhalts der Komponenten an die zentrale Verarbeitungseinheit (40).

6. Verfahren nach Anspruch 4, ferner umfassend ein Spülen des abgedichteten Gehäuses (22) nach Abschluss mindestens der Schritte von a) bis j).

7. Verfahren nach Anspruch 5, ferner umfassend das Spülen des abgedichteten Gehäuses (22) nach Abschluss mindestens der Schritte von a) bis j).

8. Verfahren nach Anspruch 4, wobei die Schritte g) und h) vorgenommen werden, durch Versehen der Vorrichtung für die Analyse von ausgeatmetem Gas mit einem Satz auswechselbarer optischer Filter-/Wellenbandsensor-Baugruppen (38), die in dem abgedichteten Gehäuse (22) in einer Position entfernbar installierbar sind, die das Ausführen der Schritte h) und i) ermöglicht.

9. Verfahren nach Anspruch 5, wobei die Schritte h) und i) vorgenommen werden, durch Versehen der Vorrichtung für die Analyse von ausgeatmetem Gas mit einem Satz auswechselbarer optischer Filter/Wellenbandsensor-Baugruppen (38), die in dem abgedichteten Gehäuse (22) in einer Position entfernbar installierbar sind, die das Ausführen der Schritte h) und i) ermöglicht.

10. Verfahren nach Anspruch 6, wobei die Schritte g) und h) vorgenommen werden, durch Versehen der Vorrichtung für die Analyse von ausgeatmetem Gas mit einem Satz auswechselbarer optischer Filter/Wellenbandsensor-Baugruppen (38), die in dem abgedichteten Gehäuse (22) in einer Position entfernbar installierbar sind, die das Ausführen der Schritte h) und i) ermöglicht.

11. Verfahren nach Anspruch 7, wobei die Schritte h) und i) vorgenommen werden, durch Versehen der Vorrichtung für die Analyse von ausgeatmetem Gas mit einem Satz auswechselbarer optischer Filter/Wellenbandsensor-Baugruppen (38), die in dem abgedichteten Gehäuse (22) in einer Position entfernbar installierbar sind, die das Ausführen der Schritte g) und h) ermöglicht.

## Revendications

1. Analyseur de contenu respiratoire portatif universel (20) comprenant :
un boîtier scellé (22) ayant une paroi extérieure, une première paroi d'extrémité (24a) et une seconde paroi d'extrémité (24b) située à l'opposé de la première paroi d'extrémité, le boîtier scellé (22) ayant une partie intérieure définie par la paroi extérieure, la première paroi d'extrémité et la seconde paroi d'extrémité ;
une première électrode (28) qui pénètre dans la partie intérieure du boîtier scellé (22) par l'une des parois d'extrémité ;
une source d'alimentation en tension connectée à la première électrode (28) pour appliquer une tension à la première électrode ;
une contre-électrode (32) destinée à interagir avec la première électrode (28) et à générer une décharge dans la partie intérieure du boîtier scellé (22) lorsqu'une tension suffisante pour générer une décharge entre la première électrode (28) et la seconde électrode (32) est appliquée à la première électrode ;
un embout buccal (30) pour prélever un échantillon de l'air exhalé par un patient et pour acheminer l'air exhalé dans la partie intérieure du boîtier scellé (22) ;
une soupape de régulation de débit (60) installée dans l'embout buccal (30) pour maintenir l'embout buccal (30) ouvert ou fermé ;
un tube d'évacuation d'air (26) inséré dans la partie intérieure du boîtier scellé (22) ;
une pompe à vide (52) avec un dispositif d'entraînement (56) connecté au tube d'évacuation d'air (26) ;
une soupape d'arrêt (54) installée dans le tube d'évacuation d'air (26) entre la partie intérieure du boîtier scellé (22) et la pompe à vide (52) ;
un capteur de pression (66) situé à l'intérieur de la partie intérieure du boîtier scellé (22) pour mesurer la pression dans ladite partie intérieure ;
une ouverture (36) dans la paroi extérieure ;
une plaque transparente (37) installée dans l'ouverture (36) ;
un jeu d'ensembles remplaçables de filtre optique/capteur à bande d'ondes (38) pouvant être installés de manière amovible sur la plaque transparente (37), chaque ensemble remplaçable de filtre optique/filtre à bande d'ondes comprenant un filtre à bande d'ondes pour faire passer la lumière de la décharge ayant une bande d'ondes prédéterminée et un capteur capable de convertir les signaux optiques de la décharge en signaux électriques ; et
une unité centrale de traitement (40), qui est connectée à la soupape de régulation de débit, à la soupape d'arrêt, à la source d'alimentation en tension, au dispositif d'entraînement (56) de la pompe à vide (52) et au capteur de pression (66) de chaque ensemble remplaçable de filtre optique/filtre à bande d'ondes, pour commander les opérations des dispositifs susmentionnés en fonction de la pression mesurée par le capteur de pression (66),
dans lequel le boîtier (22) est un corps cylindrique, au moins une partie de la paroi extérieure est transparente, et la contre-électrode (32) est un miroir semi-cylindrique situé à l'extérieur du boîtier extérieur (22) et englobant ladite au moins une partie de la paroi extérieure, qui est transparente.

2. Analyseur de contenu respiratoire portatif universel (20) selon la revendication 1, dans lequel l'unité centrale est choisie dans le groupe constitué par un ordinateur personnel, une tablette et un mobile multifonction.

3. Analyseur de contenu respiratoire portatif universel (20) selon la revendication 1, dans lequel l'unité centrale est choisie dans le groupe constitué par un ordinateur personnel, une tablette, un mobile multifonction.

4. Procédé d'analyse de gaz exhalé réalisé sous une pression constante comprenant :
a) la fourniture d'un dispositif d'analyse de gaz exhalé comprenant un boîtier scellé (22), un capteur de pression (66) pour mesurer la pression dans le boîtier scellé (22), une soupape de régulation de débit (60) pour contrôler l'admission de l'air exhalé d'un patient dans le boîtier scellé (22), une soupape d'arrêt connectée à une pompe à vide (52) pour l'évacuation de l'air du boîtier scellé (22), le boîtier scellé (22) ayant une paroi extérieure, une première paroi d'extrémité (24a) et une seconde paroi d'extrémité (24b) située à l'opposé de la première paroi d'extrémité, le boîtier scellé (22) ayant une partie intérieure définie par la paroi extérieure, la première paroi d'extrémité et la seconde paroi d'extrémité, une première électrode (28) qui pénètre dans la partie intérieure du boîtier scellé (22) à travers l'une des faces d'extrémité, et une contre-électrode (32) destinée à interagir avec la première électrode (28) et à générer une décharge dans la partie intérieure du boîtier scellé (22) lorsqu'une tension suffisante pour générer une décharge entre la première électrode (28) et la seconde électrode (32) est appliquée à la première électrode, et dans lequel le boîtier (22) est un corps cylindrique, au moins une partie de la paroi extérieure est transparente, et la contre-électrode (32) est un miroir semi-cylindrique situé à l'extérieur du boîtier extérieur (22) et englobant ladite au moins une partie de la paroi extérieure, qui est transparente ;
b) la fermeture de la soupape de régulation de débit ;
c) l'ouverture (36) de la soupape d'arrêt et l'évacuation de l'air du boîtier scellé (22) ;
d) la fermeture de la soupape d'arrêt une fois l'évacuation de l'air terminée ;
l'ouverture (36) de la soupape de régulation de débit ;
e) l'admission de l'air inhalé par un patient dans le boîtier scellé (22) tout en mesurant la pression à l'intérieur du boîtier scellé (22) ;
f) la fermeture de la soupape de régulation de débit (60) lorsque la pression mesurée par le capteur de pression (66) atteint une valeur de ladite pression constante ;
g) la génération d'une décharge dans le boîtier scellé (22) ;
h) le passage de la lumière produite par ladite décharge à travers des filtres optiques qui font passer la lumière d'une largeur de bande prédéterminée en fonction de la teneur en composants contenus dans l'air exhalé ;
i) la détection du contenu des composants ; et
j) la réalisation d'une analyse spectrale des composants contenus dans l'air exhalé par le patient dans le boîtier scellé (22).

5. Procédé selon la revendication 4, dotant en outre le dispositif d'analyse de gaz exhalé d'une unité centrale de traitement (40) ; connectant l'unité centrale de traitement (40) à la soupape de régulation de débit, à la soupape d'arrêt et au capteur de pression (66) pour commander le fonctionnement de la soupape de régulation de débit, de la soupape d'arrêt et du capteur de pression (66), en maintenant la pression à l'intérieur du boîtier scellé (22) pendant ladite étape de détection du contenu des composants ; et envoyant le contenu des composants à l'unité centrale de traitement (40).

6. Procédé selon la revendication 4, comprenant en outre le balayage du boîtier scellé (22) à l'issue d'au moins les étapes a) à j).

7. Procédé selon la revendication 5, comprenant en outre le balayage du boîtier scellé (22) à l'issue d'au moins les étapes a) à j).

8. Procédé selon la revendication 4, dans lequel les étapes g) et h) sont réalisées en fournissant au dispositif d'analyse de gaz exhalé un jeu d'ensembles remplaçables de filtre optique/capteur à bande d'ondes (38) pouvant être installés de manière amovible dans le boîtier scellé (22) dans une position permettant d'exécuter les étapes h) et i).

9. Procédé selon la revendication 5, dans lequel les étapes h) et i) sont réalisées en fournissant au dispositif d'analyse de gaz exhalé un jeu d'ensembles remplaçables de filtre optique/capteur à bande d'ondes (38) pouvant être installés de manière amovible dans le boîtier scellé (22) dans une position permettant d'exécuter les étapes h) et i).

10. . Procédé selon la revendication 6, dans lequel les étapes g) et h) sont réalisées en fournissant au dispositif d'analyse de gaz exhalé un jeu d'ensembles remplaçables de filtre optique/capteur à bande d'ondes (38) pouvant être installés de manière amovible dans le boîtier scellé (22) dans une position permettant d'exécuter les étapes h) et i).

11. Procédé selon la revendication 7, dans lequel les étapes h) et i) sont réalisées en fournissant au dispositif d'analyse de gaz exhalé un jeu d'ensembles remplaçables de filtre optique/capteur à bande d'ondes (38) pouvant être installés de manière amovible dans le boîtier scellé (22) dans une position permettant d'exécuter les étapes g) et h).
